# EUROPEAN PATENT SPECIFICATION

(11) **EP 2 560 592 B1**
(45) Date of publication and mention of the grant of the patent: **05.08.2015**
(21) Application number: 11717106.6
(22) Date of filing: 14.04.2011
(51) Int. Cl.: A61F 13/02

(54) **SYSTEMS, APPARATUSES, AND METHODS FOR SIZING A SUBCUTANEOUS, REDUCED-PRESSURE TREATMENT DEVICE**
SYSTEME, VORRICHTUNGEN UND VERFAHREN ZUR GRÖSSENEINSTELLUNG EINER SUBKUTANEN BEHANDLUNGSVORRICHTUNG MIT REDUZIERTEM DRUCK
SYSTÈMES, APPAREILS ET PROCÉDÉS DE DIMENSIONNEMENT D'UN DISPOSITIF DE TRAITEMENT À PRESSION RÉDUITE, SOUS-CUTANÉ

(30) Priority: 12.04.2011 US 201113084733; 16.04.2010 US 325128 P
(43) Date of publication of application: 27.02.2013
(73) Proprietor: KCI Licensing, Inc., San Antonio, TX 78265-9508 (US)
(72) Inventor: LOCKE, Christopher Brian, Bournemouth Dorset BH9 35D (GB); ROBINSON, Timothy Mark, Basingstoke Hampshire RG23 8HH (GB); FLOWER, Kingsley Robert George, Ringwood Hampshire BH24 1XY (GB)
(74) Representative: Cordina, Kevin John
(86) International application number: PCT/US2011/032550
(87) International publication number: WO 2011/130551

(56) References cited:
- US-A1- 2010 069 829

## Description

### RELATED APPLICATION

The present invention claims the benefit, under 35 USC § 119(e), of the filing of U.S. Provisional Patent Application serial number 61/325,128, entitled "Systems, Apparatuses, and Methods for Sizing a Subcutaneous, Reduced-Pressure Treatment Device," filed April 16, 2010, which is incorporated herein by reference for all purposes.

### BACKGROUND

The present disclosure relates generally to medical treatment systems and, more particularly, but not by way of limitation, to system, apparatuses, and methods for sizing a subcutaneous, reduced-pressure treatment device.

Whether the etiology of a wound, or damaged area of tissue, is trauma, surgery, or another cause, proper care of the wound, or wounds, is important to the outcome. Unique challenges exist when the wound involves locations that require reentry, for example, the peritoneal cavity and more generally the abdominal cavity. Often times when surgery or trauma involves the abdominal cavity, establishing a wound management system that facilitates reentry, allows for better and easier care, and helps to address such things as peritonitis, abdominal compartment syndrome, and infections that might inhibit final healing of the wound and the internal organs. In providing such care, it may be desirable to remove unwanted fluids from the cavity, help approximate the fascia and other tissues, or to help provide a closing force on the wound itself at the level of the epidermis. Unless otherwise indicated, as used throughout this document, "or" does not require mutual exclusivity.

Currently, an abdominal opening on the epidermis may be closed using sutures, staples, clips, and other mechanical devices to allow the skin, or epidermis, to be held and pulled. Such devices cause wounds in and of themselves. Moreover, without more, if edema occurs, tremendous pressure may be placed on the closure device with potential harm resulting. For example, if pressure rises due to edema, sutures may tear out.

With respect to an overall system for allowing reentry into the abdominal cavity, a number of techniques have been developed. One approach is to place towels down into the cavity and then use clips, such as hemostats, to close the skin over the towels. While simple and fast, the results appear to have been regarded as suboptimal. Another approach is the "Bogota bag." With this approach, a bag is sutured into place to cover the open abdomen. Still another approach, sometimes called a "vac pack," has been to pack towels in the wound and then place a drain into the abdomen and cover the abdomen with a drape. Finally, a reduced pressure approach has been used. Such an approach is shown in U.S. Patent 7,381,859 to Hunt et al. and assigned to KCl Licensing, Inc. of San Antonio, Texas. U.S. Patent 7,381,859 is incorporated herein by reference for all purposes.

In addition to accessing the cavity for reentry, it is often desirable to remove fluids from the cavity. It may also be desirable to provide reduced-pressure therapy to the tissue or wound, including wounds that may be within the abdominal cavity. This treatment (frequently referred to in the medical community as "negative pressure wound therapy," "reduced pressure therapy," or "vacuum therapy") may provide a number of benefits, including faster healing.

US2010/069829 discloses a wound treatment device for the application of reduce-pressure to a wound comprising a manifold manifold member encapsulated within two encapsulating members.

### SUMMARY

According to one illustrative embodiment, a method of sizing a reduced-pressure treatment device for placement within a body cavity of a patient includes providing the reduced-pressure treatment device for disposing within the body cavity. The reduced-pressure treatment device may include a manifold member, a first encapsulating member, and a second encapsulating member. The first encapsulating member and second encapsulating member encapsulate the manifold member. The reduced-pressure treatment device also includes a first reduced-pressure interface fluidly coupled to the manifold member for delivering reduced pressure to the manifold member. The method of sizing a reduced-pressure treatment device further includes sizing the reduced-pressure treatment device to form a fitted treatment device that fits the body cavity using a sizing tool. The sizing step comprises sealing the treatment device along an adjustment edge and cutting the treatment device along the adjustment edge.

According to another illustrative embodiment, a method of providing reduced-pressure treatment in a body cavity of a patient includes providing a reduced-pressure treatment device. The treatment device may include a manifold member, a first encapsulating member, and a second encapsulating member. The first encapsulating member and second encapsulating member encapsulate the manifold member. The treatment device further includes a first reduced-pressure interface fluidly coupled to the manifold member for delivering reduced pressure to the manifold member. The method of providing reduced-pressure treatment further includes sizing the treatment device using a sizing tool to form a fitted treatment device that fits the body cavity. The sizing step comprises: sealing the treatment device along an adjustment edge and cutting the treatment device along the adjustment edge. Additionally, the method of providing reduced-pressure treatment may include placing a portion of the fitted treatment device proximate a paracolic gutter in the body cavity (when the body cavity is an abdominal cavity), fluidly coupling a reduced-pressure source to the manifold member, and placing a sealing member over the treatment device and on a portion of a patient's epidermis to form a fluid seal over the body cavity.

According to another illustrative embodiment, an open-cavity, reduced-pressure treatment system for providing reduced-pressure treatment within a body cavity of a patient includes a treatment device for disposing within the body cavity. The treatment device may include a plurality of encapsulated members, each encapsulated member having a manifold member, a first encapsulating member, and a second encapsulating member. Fenestrations are formed on the second encapsulating member. The first encapsulating member and second encapsulating member encapsulate the manifold member. The treatment device may further include a first reduced-pressure interface fluidly coupled to the manifold member for delivering reduced pressure to the manifold member. The reduced-pressure treatment system further includes a sizing tool for substantially sealing and cutting the treatment device. The sizing tool is adapted to size the treatment device to form a fitted treatment device. The reduced-pressure system may further include a sealing member for disposing on a portion of a patient's epidermis and adapted to form a fluid seal over the treatment device and the body cavity, a reduced-pressure delivery conduit, a reduced-pressure source fluidly coupled to the reduced-pressure delivery conduit, and a second reduced-pressure interface for coupling to the sealing member and adapted to fluidly couple the reduced-pressure delivery conduit to the first reduced-pressure interface.

Other features and advantages of the illustrative embodiments will become apparent with reference to the drawings and detailed description that follow.

### BRIEF DESCRIPTION OF THE DRAWINGS

FIGURE 1 is a schematic, perspective view of a reduced-pressure treatment device being sized with an illustrative embodiment of a sizing tool;
FIGURE 2A is a schematic diagram, with a portion in cross section, of an illustrative embodiment of a reduced-pressure treatment device and system;
FIGURE 2B is a schematic cross section of a portion of the treatment device of FIGURE 2A;
FIGURE 2C is a schematic cross section of a portion of the treatment device of FIGURE 2A taken along line 2C-2C;
FIGURE 2D is a schematic cross section of a portion of the system of FIGURE 2A;
FIGURE 3 is a schematic, plan view of another illustrative embodiment of a reduced-pressure treatment device;
FIGURE 4A is a schematic, plan view of an illustrative embodiment of a sizing tool;
FIGURE 4B is a schematic, top view of the sizing tool of FIGURE 4A;
FIGURE 5 is a schematic cross section of a portion of the treatment device of FIGURE 2A after being sized;
FIGURE 6A is a schematic, cross-section of another illustrative embodiment of a portion of a reduced-pressure treatment device before being sized; and
FIGURE 6B is a schematic, cross-section of the illustrative embodiment of a portion of a reduced-pressure treatment device of FIGURE 6A after being sized.

### DETAILED DESCRIPTION OF ILLUSTRATIVE EMBODIMENTS

In the following detailed description of the illustrative embodiments, reference is made to the accompanying drawings that form a part hereof. These embodiments are described in sufficient detail to enable those skilled in the art to practice the invention, and it is understood that other embodiments may be utilized and that logical structural, mechanical, electrical, and chemical changes may be made without departing from the spirit or scope of the invention. To avoid detail not necessary to enable those skilled in the art to practice the embodiments described herein, the description may omit certain information known to those skilled in the art. The following detailed description is, therefore, not to be taken in a limiting sense, and the scope of the illustrative embodiments are defined only by the appended claims.

### A. INTRODUCTION

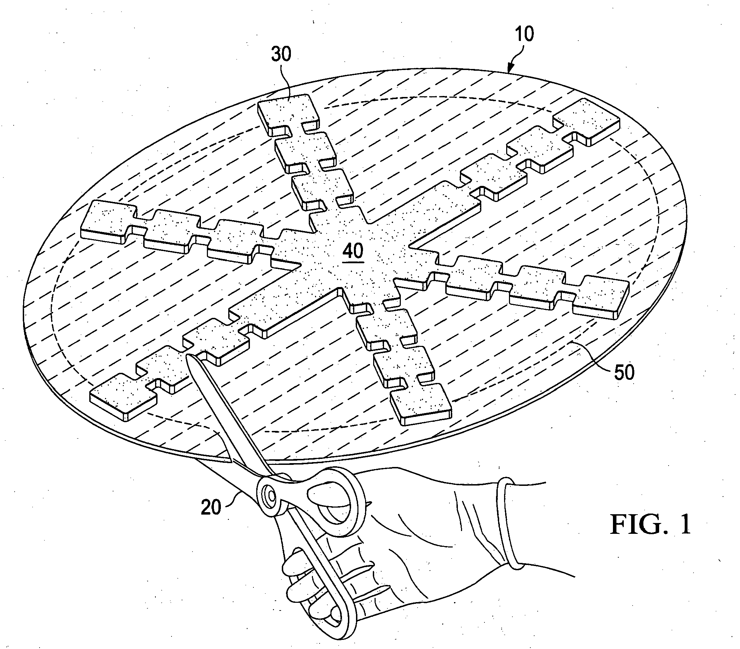

Referring now to the drawings and initially to FIGURE 1, an illustrative embodiment of a treatment device 10 being sized with a sizing tool 20 is shown. The treatment device 10 is for treating a tissue site (not shown) of a patient with reduced pressure, typically to remove fluids. The treatment device 10 may be any device containing a manifold in a drape envelope for removing fluids. For example, in the illustrative embodiment of FIGURE 1, the treatment device 10 includes a plurality of encapsulated members 30 fluidly coupled to a central connection member 40. The sizing tool 20 is for sizing the treatment device 10 to form a fitted treatment device that fits the dimensions of the tissue site being treated. The sizing tool 20 may simultaneously seal and cut the plurality of encapsulated members 30. Illustrative, non-limiting embodiments of the treatment device 10 will primarily be presented in section B and the sizing tool 20 will be described in further detail below in section C.

### B. ILLUSTRATIVE TREATMENT DEVICES AND SYSTEMS

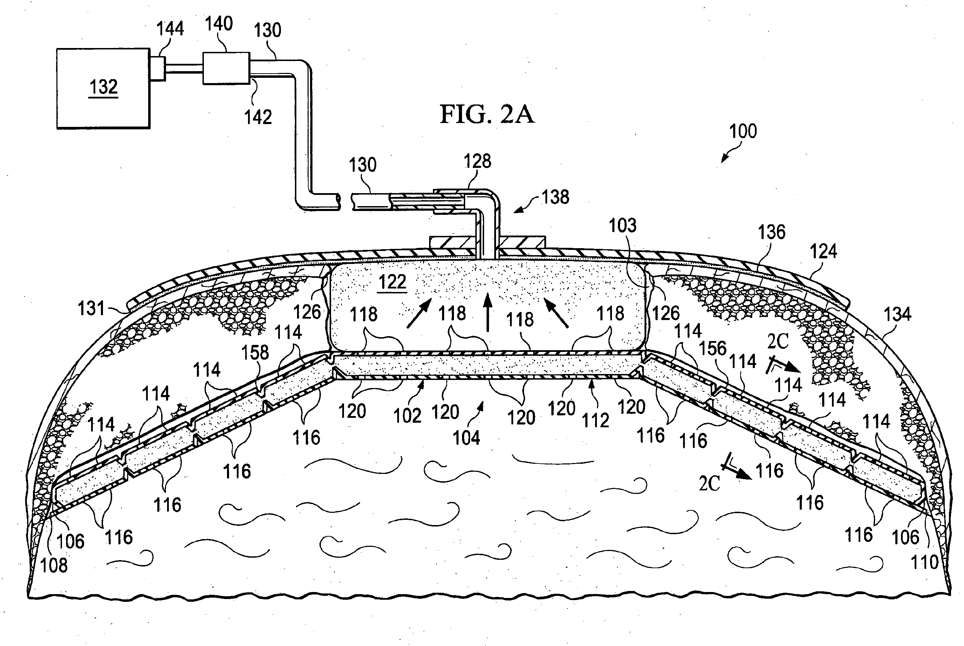

Referring now primarily to FIGURES 2A-2D, an illustrative embodiment of a reduced-pressure system 100, which may be used with an open body cavity, and a treatment device 102 are presented. The open-cavity reduced-pressure system 100 and the treatment device 102 are for treating a tissue site 104 of a patient. The tissue site 104 may be the bodily tissue of any human, animal, or other organism, including bone tissue, adipose tissue, muscle tissue, dermal tissue, connective tissue, cartilage, tendons, ligaments, or any other tissue. The tissue site may be any body cavity, but is presented in the context of the abdominal cavity 103. The abdominal cavity 103 includes the abdominal contents or tissue that is proximate the abdominal cavity 103. Treatment of the tissue site 104 may include removal of fluids, e.g., ascites, protection of the abdominal cavity 103, or reduced-pressure therapy.

As shown, the treatment device 102 is disposed within the abdominal cavity 103 of the patient to treat the tissue site 104. The treatment device 102 may include a manifold of any shape, and this embodiment includes a plurality of encapsulated members 106 that are supported by the abdominal contents, which make up a surface on which the plurality of encapsulated members 106 are placed. One or more of the plurality of encapsulated members 106 may be placed in or proximate to a first paracolic gutter 108, and one or more of the plurality of encapsulated members 106 may be placed in or proximate to a second paracolic gutter 110. The plurality of encapsulated members 106 is coupled to a central connection member 112, and there is fluid communication between the plurality of encapsulated members 106 and the central connection member 112. The plurality of encapsulated members 106 or the central connection member 112 may be formed with fenestrations 114, 116, 118, 120 that allow fluids in the abdominal cavity 103 to pass through. The fenestrations 114, 116, 118, 120 may take any shape, e.g., circular apertures, rectangular openings, or polygons, and are presented in this illustrative embodiment as slits, or linear cuts. One or more fenestrations 114, 116, 118, 120 may be omitted in alternative embodiments.

A system manifold 122, or first reduced pressure interface, is fluidly coupled to the encapsulated members 106 and distributes reduced pressure to the treatment device 102. A sealing member 124 provides a fluid seal over a body-cavity opening 126. "Fluid seal," or "seal," means a seal adequate to maintain reduced pressure at a desired site given the particular reduced-pressure source or subsystem involved. One or more skin closure devices may be placed on a patient's epidermis 134. Reduced pressure is delivered to the system manifold 122 through a reduced-pressure interface 128, or a second reduced-pressure interface, which is fluidly coupled to a reduced-pressure delivery conduit 130 and to the system manifold 122. A reduced-pressure source 132 delivers reduced pressure to the reduced-pressure delivery conduit 130.

The reduced pressure may be applied to the tissue site 104 to help promote removal of ascites, exudates, or other fluids from the tissue site 104. In some instances, reduced pressure may be applied to stimulate the growth of additional tissue. In some instances, only fluid removal may be desired. In the case of a wound at the tissue site 104, the growth of granulation tissue, removal of exudates, or removal of bacteria may help to promote healing of the wound. As used herein, "reduced pressure" generally refers to a pressure less than the ambient pressure at a tissue site that is being subjected to treatment. In most cases, this reduced pressure will be less than the atmospheric pressure at which the patient is located. Alternatively, the reduced pressure may be less than a hydrostatic pressure at the tissue site 104. Unless otherwise indicated, values of pressure stated herein are gauge pressures. The reduced pressure delivered may be constant or varied (patterned or random) and may be delivered continuously or intermittently. Consistent with the use herein, unless otherwise indicated, an increase in reduced pressure or vacuum pressure typically refers to a relative reduction in absolute pressure.

The system manifold 122 (or first reduced pressure interface) is disposed proximate the central connection member 112. The system manifold 122, or first reduced-pressure interface, may take many forms. For example, the system manifold 122 may be a manifolding material. The term "manifold" as used herein generally refers to a substance or structure that is provided to assist in applying reduced pressure to, delivering fluids to, or removing fluids from the tissue site 104. The system manifold 122 typically includes a plurality of flow channels or pathways that distribute the fluids provided to and removed from the tissue site 104 around the system manifold 122 and through the central connection member 112. In one illustrative embodiment, the flow channels or pathways are interconnected to improve distribution of fluids provided or removed from the tissue site 104. The system manifold 122 may be a biocompatible material that is capable of being placed in contact with tissue.

Examples of the system manifold 122 may include, without limitation, devices that have structural elements arranged to form flow channels, cellular foam, such as open-cell foam, reticulated foam, porous tissue collections, liquids, gels and foams that include or cure to include flow channels. The system manifold 122 may be porous and may be made from foam, gauze, felted mat, or any other material suited to a particular biological application.

In one embodiment, the system manifold 122 is a porous foam and includes a plurality of interconnected cells or pores that act as flow channels. The porous foam may be a polyurethane, open-cell, reticulated foam, such as a GranuFoam® material manufactured by Kinetic Concepts, Incorporated of San Antonio, Texas. Other embodiments may include "closed cells" to control flow. In some situations, the system manifold 122 may also be used to distribute fluids such as medications, antibacterials, growth factors, and various solutions to the tissue site 104. Other layers may be included in or on the system manifold 122, such as absorptive materials, wicking materials, hydrophobic materials, and hydrophilic materials.

The system manifold 122, or first reduced-pressure interface, may also be any device that operates to deliver reduced pressure to the central connection member 112. For example, in one illustrative embodiment, the system manifold 122 may be one or more fluid delivery conduits (not shown) that enter the abdominal cavity 103 and fluidly couple to the central connection member 112. In other words, a conduit may be coupled to a reduced-pressure source external to the patient and the conduit may be fluidly coupled to the central connection member 112, which may have a fitting to receive the conduit or may directly receive the conduit into the central connection member 112. Typically for the embodiment shown, however, the system manifold 122 is a polymer foam that transmits reduced pressure to the central connection member 112.

The sealing member 124 is placed over the body-cavity opening 126 and provides a fluid seal adequate for the open-cavity, reduced-pressure system 100 to hold a reduced pressure at the tissue site 104. The sealing member 124 may be a cover that is used to secure the system manifold 122 on the central connection member 112. The sealing member 124 may be impermeable or semi-permeable. The sealing member 124 is capable of maintaining reduced pressure at the tissue site 104 after installation of the sealing member 124 over the body-cavity opening 126. The sealing member 124 may be a flexible over-drape or film formed from a silicone-based compound, acrylic, hydrogel or hydrogel-forming material, or any other biocompatible material that includes impermeability or permeability characteristics as desired for applying reduced pressure to the tissue site 104.

The sealing member 124 may further include an attachment device 131 to secure the sealing member 124 to the patient's epidermis 134. The attachment device 131 may take many forms. For example, the attachment device 131 may be an adhesive layer 136 that is positioned along a perimeter of the sealing member 124 or any portion of the sealing member 124 to provide, directly or indirectly, the fluid seal with the patient's epidermis 134. The adhesive layer 136 may also be pre-applied to the sealing member 124 and covered with a releasable backing, or member (not shown), that is removed at the time of application.

The reduced-pressure interface 128 may be, as one example, a port or connector 138, which permits the passage of fluid from the system manifold 122, to the reduced-pressure delivery conduit 130 and vice versa. For example, fluid collected from the tissue site 104 using the system manifold 122 and the treatment device 102 may enter the reduced-pressure delivery conduit 130 via the connector 138. In another embodiment, the open-cavity, reduced-pressure system 100 may omit the connector 138 and the reduced-pressure delivery conduit 130 may be inserted directly into the sealing member 124 and may be inserted into the system manifold 122.

The reduced-pressure delivery conduit 130 may be a medical conduit or tubing or any other means for transporting a reduced pressure and fluid. The reduced-pressure delivery conduit 130 may be a multi-lumen member for readily delivering reduced pressure and removing fluids. In one embodiment, the reduced-pressure delivery conduit 130 is a two-lumen conduit with one lumen for reduced pressure and liquid transport and one lumen for communicating pressure to a pressure sensor.

Reduced pressure is supplied to the reduced-pressure delivery conduit 130 by the reduced-pressure source 132. A wide range of reduced pressures may be supplied by the reduced-pressure source 132. In one embodiment, the range may include the range of -50 to-300 mm Hg and in another embodiment, the range may include -100 mm Hg (-13.3 kPa) to-200 mm Hg (-26.6 kPa). For example, and not by way of limitation, the pressure may be -12, -12.5, -13, -14, -14.5, -15, -15.5, -16, -16.5, -17, -17.5, -18, -18.5, -19, -19.5, -20, -20.5, -21, - 21.5, -22, -22.5, -23, -23.5, -24, -24.5, -25, -25.5, -26, -26.5 kPa or another pressure. In one illustrative embodiment, the reduced-pressure source 132 includes preset selectors for -100 mm Hg (-13.3 kPa), -125 mm Hg (-16.6 kPa), and -150 mm Hg (-19.9 kPa). The reduced-pressure source 132 may also include a number of alarms, such as a blockage alarm, a leakage alarm, or a battery-low alarm. The reduced-pressure source 132 may be a portable source, wall source, or other unit for abdominal cavities. The reduced-pressure source 132 may selectively deliver a constant pressure, varied pressure (random or patterned), intermittent pressure, or continuous pressure. The fluid removed from the cavity through the reduced-pressure delivery conduit 130 could be as much as 5L or more per day for some applications.

A number of different devices, e.g., device 140, may be added to a medial portion 142 of the reduced-pressure delivery conduit 130. For example, the device 140 may be a fluid reservoir, or canister collection member, a pressure-feedback device, a volume detection system, a blood detection system, an infection detection system, a filter, a port with a filter, a flow monitoring system, or a temperature monitoring system. Multiple devices 140 may be included. Some of these devices, e.g., the fluid collection member, may be formed integrally with the reduced-pressure source 132. For example, a reduced-pressure port 144 on the reduced-pressure source 132 may include a filter member (not shown) that includes one or more filters and may include a hydrophobic filter that prevents liquid from entering an interior space of the reduced-pressure source 132.

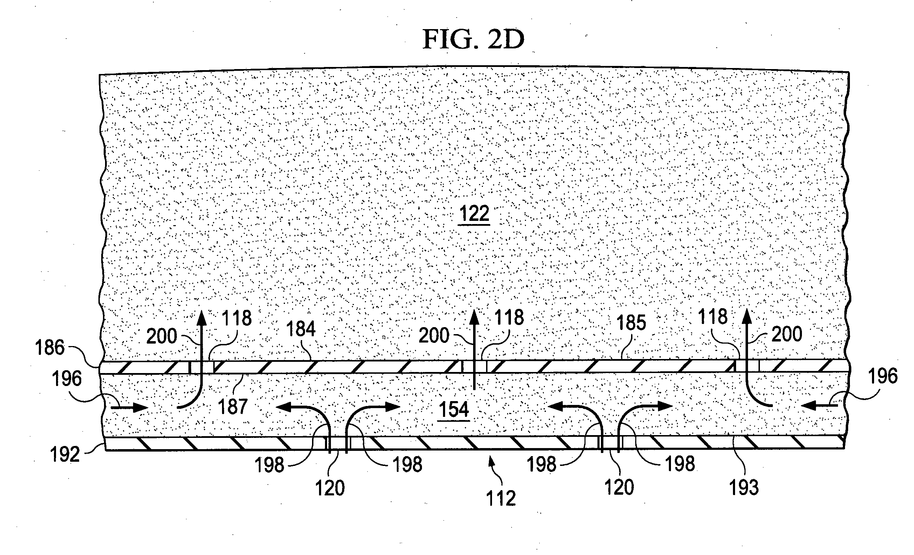

As shown, the treatment device 102 includes the central connection member 112 to which the plurality of encapsulated members 106 are coupled. Referring primarily to FIGURE 2D, the central connection member 112 includes a connection manifold member 154 that is encapsulated by a first connection encapsulation member 186 and a second connection encapsulation member 192, which is a patient-facing encapsulation member. The central connection member 112 is fluidly coupled to the plurality of encapsulated members 106. The first and second connection encapsulation members 186, 192 may be a defined by a single piece of material or, as illustrated, more than one sheet of material.

The central connection member 112 fluidly communicates with the system manifold 122. In one aspect, the fenestrations 118, similar to the fenestrations discussed above, can permit fluid communication.

Referring again primarily to FIGURES 2A-2D, each of the plurality of encapsulated members 106 may include a manifold member 160, which may be a single manifold member that runs along the plurality of encapsulated members 106 to the central connection member 112, or individual manifold components. The manifold member 160 may be formed from the same types of materials that the system manifold 122 may be formed. The manifold member 160 is disposed within an interior portion 162 of each of the encapsulated members 106. Each manifold member 160 has a first side 164 and a second, inward-facing (patient-facing) side 166. The manifolds 122, 160 may each be formed as an integral member or from separate members.

In one embodiment, one or more of the plurality of manifold members 160 may have different material properties or structures. For example, different flow rates may be desired in different encapsulated members 106. Different manifold materials or manifold properties, different manifold sizes, manifold compression, flow restricting material structures, or valves may be used to provide different flow rates through the encapsulated members 106 or central connection member 112.

A first encapsulating member 168, having a first side 188 and a second, patient-facing side 189, which may be formed with fenestrations 114, is disposed on the first side 164 of the manifold member 160. A second encapsulating member 170, having a first side 190 and a second, patient-facing side 191, which may include fenestrations 116, is disposed on the second, inward-facing side 166 of the manifold member 160.

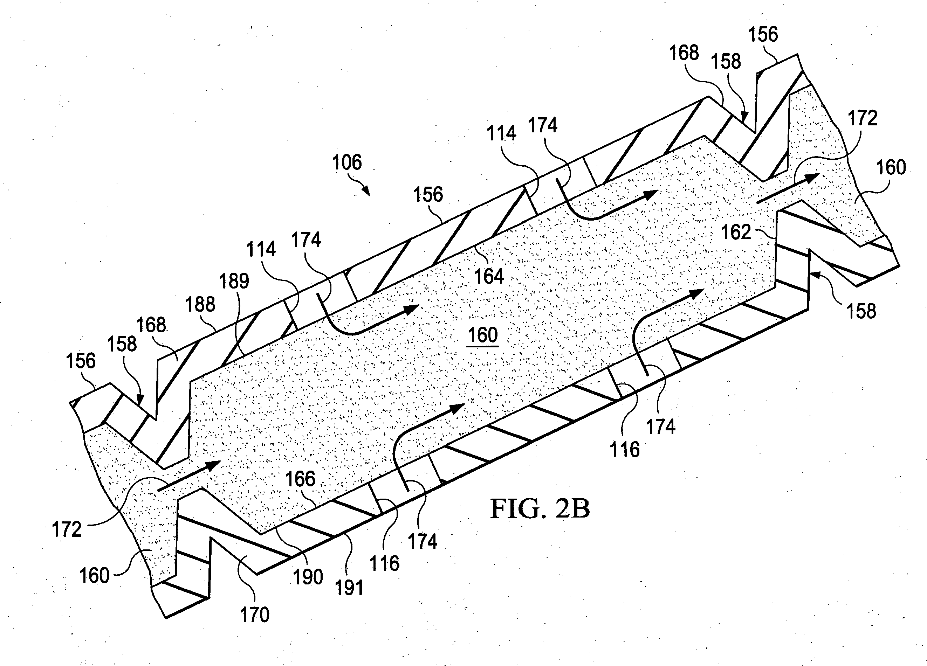

As shown in the longitudinal cross section of FIGURE 2B by arrows 172, fluid can flow in the encapsulated members 106 towards the central connection member 112. As shown by arrows 174, the fluid at or near the tissue site 104 is able to enter fenestrations 114 and 116 and flow into the manifold member 160 and then flow toward the central connection member 112 as represented by arrows 172.

In plan view, the encapsulated members 106 may take a number of different shapes, such as elongate shapes, rectangular, elliptical, or any other shape. The encapsulated members 106 may include leg modules 156. Adjacent leg modules 156 are fluidly coupled to each other and may have manipulation zones 158 between them. The manipulation zones 158 facilitate movement of the plurality of encapsulated members 106 within the body cavity. The encapsulated members 106 may also have various dimensions.

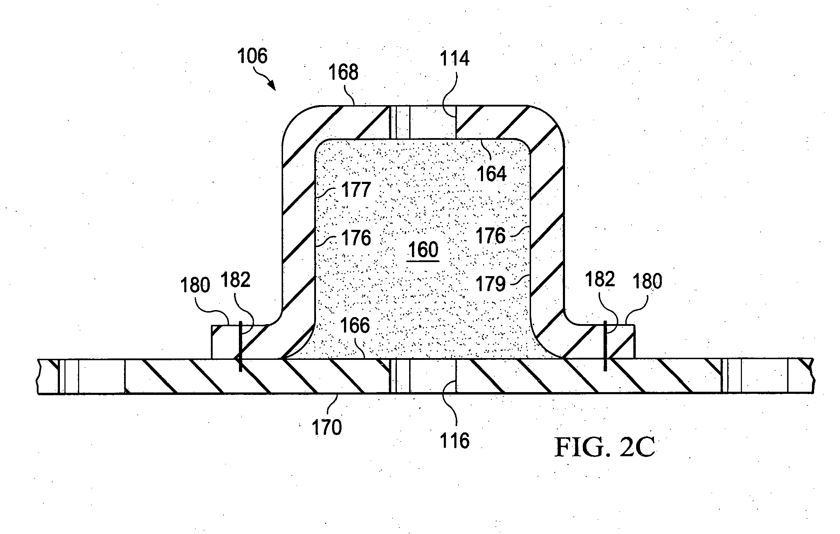

Referring now primarily to FIGURE 2C, a lateral cross section of a portion of the encapsulated member 106 is presented. As before, it can be seen that the first side 164 of the manifold member 160 is covered with the first encapsulating member 168, and that the second, inward-facing side 166 of the manifold member 160 is covered by the second encapsulating member 170. In this illustrative embodiment, a peripheral edges 176 of the manifold member 160 are also covered by a portion of the first encapsulating member 168. The peripheral edges 176 include a first lateral edge 177 and a second lateral edge 179. The first encapsulating member 168 covers the first side 164 and the peripheral edges 176 and extends onto the second encapsulating member 170 and forms extensions 180. The extensions 180 have been coupled to the second encapsulating member 170 by welds 182. The first encapsulating member 168 may, however, be coupled to the second encapsulating member 170 using any known technique, including welding (e.g., ultrasonic or RF welding), bonding, adhesives, or cements.

Referring again primarily to FIGURE 2D, the central connection member 112 includes the connection manifold member 154 that is encapsulated within the first connection encapsulation member 186, which has fenestrations 118. The first connection encapsulation member 186 is disposed proximate to a first side 187 of the connection manifold member 154. The second connection encapsulation member 192 is disposed proximate to a second, inward-facing side 193 of the connection manifold member 154. The second connection encapsulation member 192 is formed with fenestrations 120. The central connection member 112 is fluidly coupled to the encapsulated members 106. Fluid may also enter directly into the connection manifold member 154 by flowing through fenestrations 120 as suggested by arrows 198.

The system manifold 122 is disposed proximate to the first connection encapsulation member 186, and when a reduced pressure is applied to the system manifold 122, the reduced pressure causes fluid to flow from the connection manifold member 154 through fenestrations 118 and into the system manifold 122 as suggested by arrows 200. The fluid continues to flow in the direction of the reduced-pressure interface 128 through which the fluid is removed to the reduced-pressure delivery conduit 130.

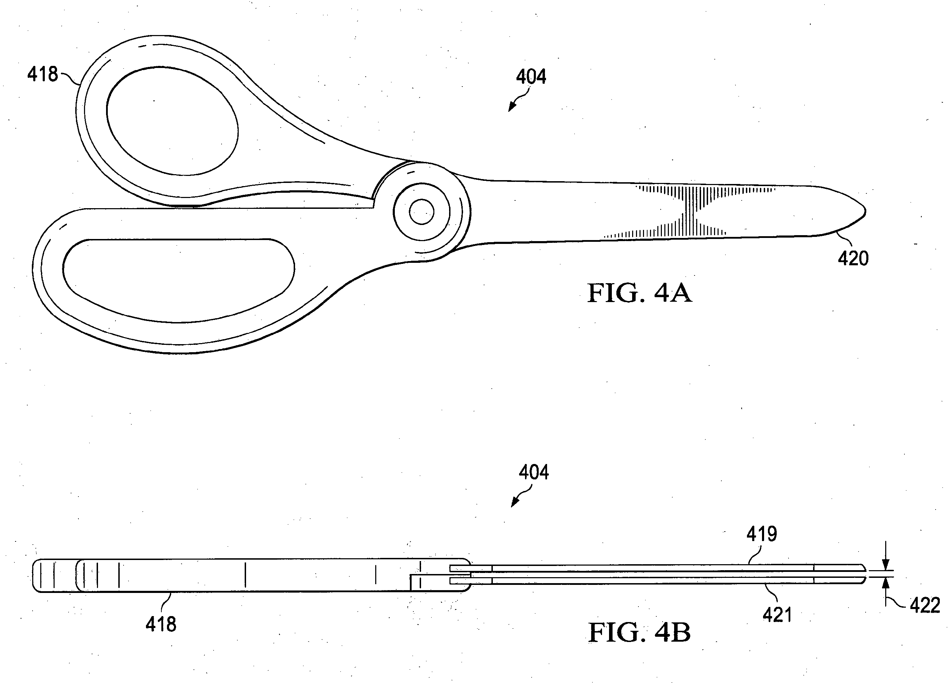

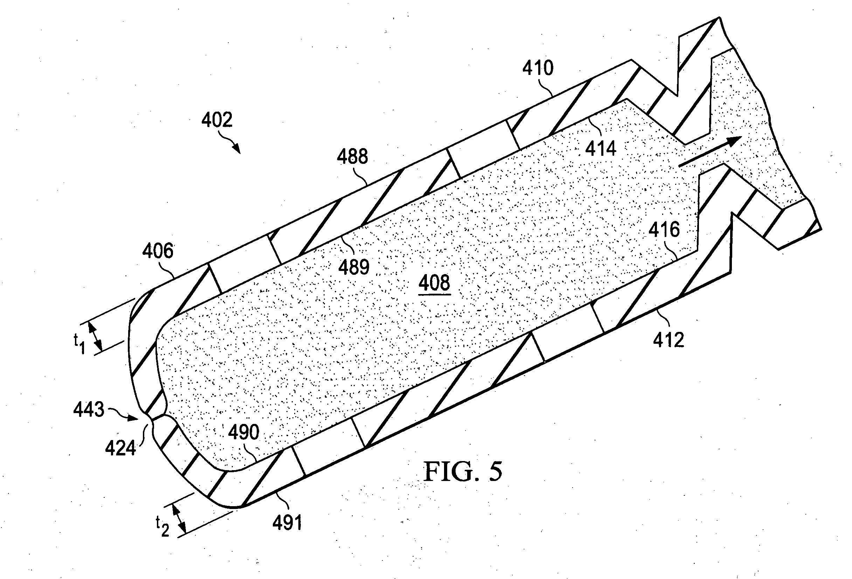

Referring now primarily to FIGURES 2A-2D, in operation according one illustrative embodiment, the illustrative open-cavity, reduced-pressure system 100 may be deployed by first sizing the treatment device 102 to form a fitted treatment device as will be explained further below in connection with FIGURES 4A-4B and 5. The plurality of encapsulated members 106 is deployed within the abdominal cavity 103 through a body-cavity opening 126 and distributed against the abdominal contents. This deployment of the encapsulated members 106 may include placing at least one encapsulated member 106 in or proximate the first paracolic gutter 108, the second paracolic gutter 110, behind the liver, or another location. Once the treatment device 102 has been deployed, the system manifold 122 is placed adjacent a first side 184 of the first connection encapsulation member 186. The sealing member 124 may then be applied over the body-cavity opening 126 to provide a fluid seal over the body-cavity opening 126.

In addition to the sealing member 124, the body-cavity opening 126 may be further closed or reinforced using mechanical closing means, e.g., staples, or using a reduced-pressure closure system. The sealing member 124 may be applied in a number of ways, and according to one illustrative embodiment, the releasable backing member that is on the adhesive layer 136 of the sealing member 124 is removed and then the sealing member 124 is placed against the patient's epidermis 134 about the body-cavity opening 126. The reduced-pressure interface 128, such as connector 138, is then attached to the sealing member 124 such that reduced pressure can be delivered by the reduced-pressure interface 128 (or second reduced-pressure interface), through the sealing member 124, and to the system manifold 122. The reduced-pressure delivery conduit 130 is fluidly coupled to the reduced-pressure interface 128 and to the reduced-pressure port 144 on the reduced-pressure source 132.

The reduced-pressure source 132 is activated and thereby provides reduced pressure into the reduced-pressure delivery conduit 130, which delivers the reduced pressure to the reduced-pressure interface 128 and into the system manifold 122. The system manifold 122 distributes the reduced pressure and draws fluid through fenestrations 118 from the connection manifold member 154. The connection manifold member 154 draws fluid from the abdominal cavity 103 through fenestrations 120 and pulls fluid from the plurality of encapsulated members 106 as suggested by arrows 196. Fluid from the abdominal cavity 103 flows into the plurality of encapsulated members 106 through fenestrations 114 on the first encapsulating member 168 and through fenestrations 116 on the second encapsulating member 170 and then flows through the plurality of encapsulated members 106 as suggested by arrows 172 towards the connection manifold member 154. The fluid then flows through the system manifold 122, the reduced-pressure interface 128 (or second reduced-pressure interface), and into the reduced-pressure delivery conduit 130.

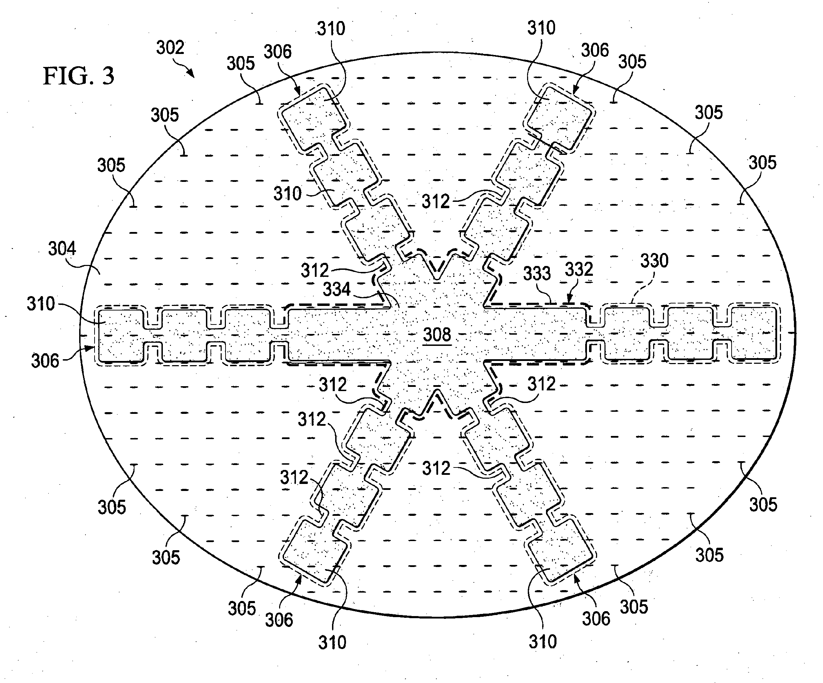

Referring now primarily to FIGURE 3, another illustrative embodiment of an open-cavity, reduced-pressure treatment device 302 is presented. The open-cavity, reduced-pressure treatment device 302 is analogous in most respects to the treatment device 102 of FIGURES 2A-2D. The open-cavity, reduced-pressure treatment device 302 includes a non-adherent drape 304. The reduced-pressure treatment device 302 has the non-adherent drape 304, a plurality of encapsulated members 306, and a central connection member 308. The central connection member 308 and the manifold members in leg modules 310 may be formed as one piece of manifold material or as a plurality of manifold members or pieces.

The non-adherent drape 304 may be formed of a non-adherent material that inhibits tissue adhesion to the non-adherent drape 304. In one embodiment, the non-adherent drape 304 is formed from a breathable polyurethane film. The non-adherent drape 304 may include a plurality of openings, apertures, or fenestrations 305. The fenestrations 305 may take a variety of shapes, such as circular openings, rectangular openings, polygon-shaped openings, or other shape. Depending on the particular application of the treatment device 302, the desired fluid flow or pressure delivery, or other system parameters, the fenestrations may be different sizes. In this particular illustrative embodiment, the non-adherent drape 304 is formed generally with an oval or arcuate shape. The non-adherent drape 304 may form at least a portion of a second encapsulating member (see by analogy the second encapsulating member 170 in FIGURE 2B) and the second connection encapsulation member (see by analogy 192 in FIGURE 2D). As such, the plurality of fenestrations 305 serves as flow channels for the plurality of encapsulated members 306 and the central connection member 308 on the second, inward-facing side. The non-adherent drape 304 may also be used on the first side of the plurality of encapsulated members 306 and the central connection member 308. In one embodiment, the encapsulated members 306 may be coupled with one another, for example, via the non-adherent drape 304. Alternatively, the encapsulated members 306 may be independently movable with respect to one another with the exception of their proximal end adjacent to the central connection member 308. For example, the encapsulated members 306 need not be connected to one another. In another embodiment, a portion of the material connecting the encapsulated members, e.g., the non-adherent drape 304 between adjacent encapsulated members 306, is expandable (e.g., a stretchable, flexible, deformable, or elastic material) and permits movement of individual encapsulated members 306 with respect to one another.

Each of the encapsulated members 306 may be formed with a plurality of leg modules 310 and with manipulation zones 312 between the plurality of leg modules 310. As with the manipulation zones 158 in FIGURES 2A-D, the manipulation zones 312 facilitate movement of the plurality of encapsulated members 306 within the body cavity.

The illustrative treatment devices 10, 102, and 302 are typically sized as an aspect of deployment within a body cavity. In sizing the treatment devices 10, 102, 302, the treatment device 10, 102, 302 is cut to an appropriate size and, typically, care is taken to present the manifold member, e.g., manifold member 160, such that the manifold member does not come in direct contact with the tissue site. A sizing tool may be used for this purpose as will now be described in more detail.

### C. ILLUSTRATIVE SIZING TOOLS AND TREATMENT DEVICES

Referring now to FIGURES 4A-4B, an illustrative embodiment of a sizing tool 404 to size a treatment device, e.g., treatment device 10, 102, 302, 402, is presented. The sizing tool 404 is for adjusting the size of the treatment device 402 along an adjustment edge (e.g., adjustment edge 50 in FIGURE 1) to fit the particular dimension of a patient's body cavity or abdominal cavity. The adjustment edge 50 defines the dimensions of the treatment device needed to substantially fit the patient's body cavity.

While the illustrative sizing tool 404 may be used with any of the treatment devices mentioned herein, or equivalents, the sizing tool will be described in connection with treatment device 402. Referring to FIGURE 5, the treatment device 402 is analogous to treatment device 102 and 302 and only a cross section of a portion is presented. The treatment device 402 is shown having been sized along an adjustment edge 443. The treatment device 402 includes one or more encapsulated members 406. Each of the encapsulated members 406 includes a manifold member 408, a first encapsulating member 410, and a second encapsulating member 412. The first encapsulating member 410 and the second encapsulating member 412 encapsulate the manifold member 408.

The manifold member 408 has a first side 414, a second, inward-facing side 416, and a first and second lateral edge (not shown). The first encapsulating member 410 has a first side 488 and a second, patient-facing side 489. The second encapsulating member 412 has a first side 490 and a second-patient facing side 491. The second, patient-facing side 489 of the first encapsulating member 410 is disposed proximate the first side 414 of the manifold member 408 and the first side 490 of the second encapsulating member 412 is disposed proximate the second, inward-facing side 416 of the manifold member 408. The first encapsulating member 410 and the second encapsulating member 412 are coupled proximate the first and second lateral edge of the manifold member 408.

Referring again to FIGURES 4A-4B, the sizing tool 404 includes a sealing-and-cutting implement or scissors 418 having a first blade 419 and a second blade 421 with a blade clearance 422, or blade gap, between the blades 419, 421. The blade clearance 422 depends on the thickness of the encapsulating members 410, 412 and the compressed thickness of the manifold member 408. In an illustrative embodiment, the scissors 418 may have a blade clearance 422 in the rage of 0.1-0.6 mm and in one embodiment may be approximately 0.3 mm. If the first encapsulating member 410 has a first thickness (t₁), the second encapsulating member 412 has a second thickness (t₂), and the manifold member 408 has a compressed thickness (t₃), the blade clearance (bc) range may be sized such that bc < 0.9 * (t₁+t₂+t₃) and bc > 0.1*(t₁+t₂+t₃). As an illustrative, non-limiting specific example, if t₁ = 50 microns, t₂ = 50 microns, and t₃ = 3 millimeters (mm), then bc > 0.31 mm and < 2.79 mm.

The step of sizing the encapsulated member 406 includes crimping or otherwise sealing and cutting simultaneously the encapsulated member 406 along an adjustment edge. The cutting edges 420 of the scissors 418 with the blade clearance 422 function to crimp or compress the encapsulated member 406 along the adjustment edge and then cut or tear the encapsulated member 406. As used herein, "simultaneously," means occurring at substantially the same time. The sizing step cuts the treatment device and creates a bond 424 between the first and second encapsulating members 410, 412. The bond 424 may be caused by friction or friction cut between the blades 419, 421. The bond 424 substantially forms a seal between the first and second encapsulating members 410, 412 along the adjustment edge. Functionally, the sizing tool 404 cuts and seals the encapsulating members 410, 412. Other embodiments of the sizing tool 404 and treatment device 402 may cooperate to cut and seal the treatment device 402.

Alternatively, illustrative embodiments of the sizing tool 404 (not shown) may include, for example, spring-loaded blades. The spring-loaded blades make up a sizing tool that compresses the treatment device 402 to a critical compressive force to seal and then cuts or tears the treatment device 402 once the critical compressive force (i.e., at least adequate to substantially seal) is achieved. The approach of sequentially compressing and cutting is regarded as simultaneous because the steps occur substantially at the same time.

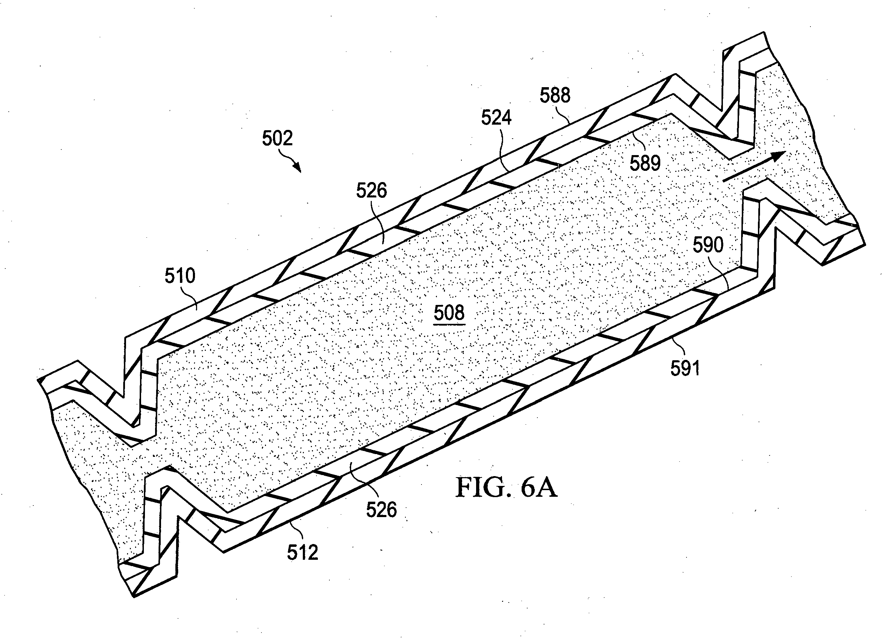

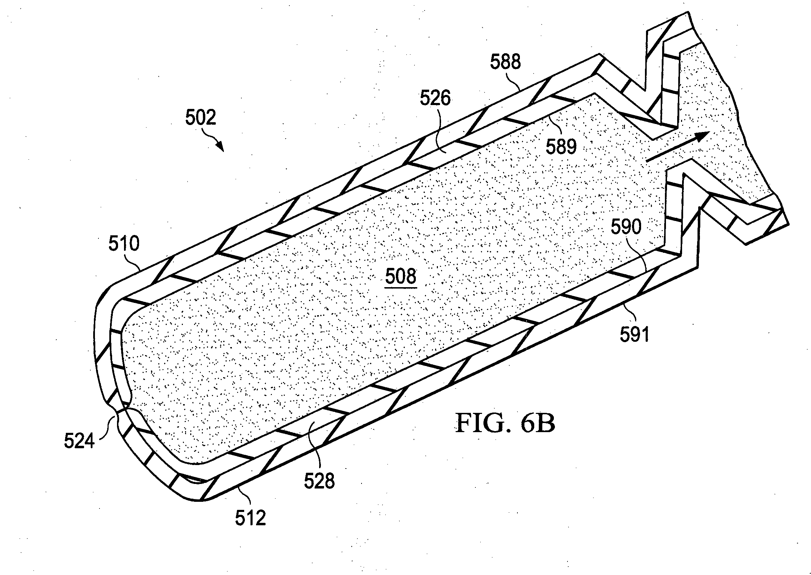

Referring now to FIGURES 6A and 6B, an alternative, illustrative embodiment of a treatment device 502 that has been sized is presented. The treatment device 502 is similar to treatment device 402 of FIGURE 5, and to indicate analogous parts, reference numerals have been indexed by 100. The treatment device 502 includes a manifold member 508, a first encapsulating member 510, and a second encapsulating member 512. The treatment device 502 differs primarily in that a second, patient-facing side 589 of a first encapsulating member 510 includes a first bonding layer 526 and a first side 590 of a second encapsulating member 512 includes a second bonding layer 528.

In an illustrative embodiment, the first and second bonding layers 526, 528 may be chemical bond layers. The chemical bond layers are formed from reactive materials such that when the first bonding layer 526 is brought into contact with the second bonding layer 528, via the sizing step, a chemical reaction occurs that forms a bond 524. The sizing step substantially seals the manifold member 508 by creating the bond 524, which is a chemical bond. A color indicator may be included in the first and second bonding layers 526, 528 that becomes active when the first and second bonding layers 526, 528 create the bond 524. The first and second bonding layers 526, 528 may be formed, for example, by using a two-part chemical reaction system. Two-part chemical reaction systems may include an epoxy, where one part may contains an epoxide groups attached to a polymer, and the other part contains a polymer with amine functional groups; a Silicone, where one part contains a silicone polymer with platinum catalyst, and the other part contains a silicone polymer with a vinyl group; an ionic, where one part contains a polymer with carboxylic groups, and the other part contains a polymer with hydroxyl groups and a ionic catalyst, such that a metal salt - polyester bonds forms; or other chemical reaction systems. The formed bond 524 may be in place of a friction bond (e.g., bond 424 in FIGURE 5) or in addition to the friction bond.

In another illustrative embodiment, the first and second bonding layers 526, 528 may be pressure-sensitive adhesive layers. The pressure-sensitive adhesive layers are formed from adhesive materials such that when the first bonding layer 526 is brought into physical contact with the second bonding layer 528, via the sizing step, a physical bond, or tacky bond, occurs to form the bond 524. The sizing step substantially seals the manifold member 508. A color indicator may be included in the first and second bonding layers 526, 528 that becomes active when the first and second bonding layers 526, 528 create the bond 524. The bond 524 may be in place of a friction bond (e.g., bond 424 in FIGURE 5) or in addition to the friction bond. Typical pressure-sensitive adhesives that be used for the pressure-sensitive adhesive layers include, without limitation, acrylic based polymers, polyurethane based polymers, and other elastomers including butylene based polymers, styrene block based polymers, silicones, ethylene, vinyl acetate, and blends and copolymers of these.

Although the present invention and its advantages have been disclosed in the context of certain illustrative, non-limiting embodiments, it should be understood that various changes, substitutions, permutations, and alterations can be made without departing from the scope of the invention as defined by the appended claims. It will be appreciated that any feature that is described in a connection to any one embodiment may also be applicable to any other embodiment.

It will be understood that the benefits and advantages described above may relate to one embodiment or may relate to several embodiments. It will further be understood that reference to 'an' item refers to one or more of those items.

The steps of the methods described herein may be carried out in any suitable order, or simultaneously where appropriate.

Where appropriate, aspects of any of the examples described above may be combined with aspects of any of the other examples described to form further examples having comparable or different properties and addressing the same or different problems.

It will be understood that the above description of preferred embodiments is given by way of example only and that various modifications may be made by those skilled in the art. The above specification, examples and data provide a complete description of the structure and use of exemplary embodiments of the invention. Although various embodiments of the invention have been described above with a certain degree of particularity, or with reference to one or more individual embodiments, those skilled in the art could make numerous alterations to the disclosed embodiments without departing from the scope of the claims.

## Claims

1. A method of sizing a reduced-pressure treatment device (10, 102, 302, 502) for placement within a body cavity of a patient, the method comprising:
providing the reduced-pressure treatment device (10, 102) for disposing within the body cavity, wherein the reduced-pressure treatment device (10, 102, 302, 502) comprises:
a manifold member (160,508),
a first encapsulating member (168, 510) having a first side (188) and a second, patient-facing side (189, 589),
a second encapsulating member (170, 304, 512) having a first side (190, 590) and a second, patient-facing side (191), wherein fenestrations (116, 305) are formed in the second encapsulating member (170),
wherein the first encapsulating member (168) and second encapsulating member (170) encapsulate the manifold member (160), and
a first reduced-pressure interface fluidly coupled to the manifold member (160, 508) for delivering reduced pressure to the manifold member (160); and
**characterised by** sizing the reduced-pressure treatment device (10, 102) to form a fitted treatment device that fits the body cavity using a sizing tool (404), the sizing step comprising:
sealing the treatment device (10, 102, 302) along an adjustment edge and simultaneously cutting the treatment device (10, 102, 302) along the adjustment edge. ,

2. The method of sizing the reduced-pressure treatment device (10, 102, 302, 502) of claim 1, wherein the steps of sealing the treatment device (10, 102, 302) and cutting the treatment device (10, 102, 302) comprise forming a friction cut.

3. The method of sizing the reduced-pressure treatment device (10, 102, 302, 502) of claims 1 or 2, wherein:
the manifold member (160, 508) has a first side, a second, inward-facing side, a first lateral edge, and a second lateral edge; and
the first encapsulating member (168, 510) is disposed proximate the first side of the manifold member (160, 508), the second encapsulating member (170, 304, 512) is disposed proximate the second, inward-facing side of the manifold member (160, 508), and the first encapsulating member (168, 510) and the second encapsulating member (170, 304, 512) are coupled proximate the first lateral edge and the second lateral edge of the manifold member (160, 508).

4. The method of sizing the reduced-pressure treatment device (502) of claim 1 or claim 3, further comprising:
a first bonding layer (526) on the second, patient-facing (589) side of the first encapsulating member (510);
a second bonding layer (528) on the first side (590) of the second encapsulating member (512); and
wherein the sizing step further comprises causing the first bonding layer (526) and the second bonding layer (528) to come into contact and form a bond.

5. The method of sizing the reduced-pressure treatment device (10, 102, 302, 502) of claim 1, 2, or 4, wherein:
the manifold member (160, 508) comprises reticulated foam and has a first side, a second, patient-facing side, a first lateral edge, and a second lateral edge;
the first encapsulating member (168, 510) is disposed proximate the first side of the manifold member (160, 508), the second encapsulating member (170, 304, 512) is disposed proximate the second, patient-facing side of the manifold member (168, 510), and the first encapsulating member (168, 510) and the second encapsulating member (170, 304, 512) are coupled proximate the first lateral edge and the second lateral edge of the manifold member (160, 508) to form an encapsulated member (106, 406);
the treatment device further comprises:
a plurality of encapsulated members (106, 406), each encapsulated member (106, 406) having fenestrations (116) to allow fluid flow between an outer surface of the encapsulated member, and
a central connection member(112), wherein the central connection member (112) comprises a connection manifold member (154) and wherein the plurality of encapsulated members (106, 406) are fluidly coupled to the connection manifold member (112), the connection manifold member (112) having a first side and a second, patient-facing side; and
the sizing step further comprises using the sizing tool (404) to cut along the adjustment edge of the reduced-pressure treatment device and to substantially seal the manifold member of the reduced-pressure treatment device within an interior portion.

6. A method of sizing the reduced-pressure treatment device (10, 102, 302, 502) of claim 1, further comprising the steps of:
placing a portion of the fitted treatment device (10, 102, 302, 502) in the body cavity;
fluidly coupling a reduced-pressure source to the manifold member (160, 508); and
placing a sealing member (124) on a portion of a patient's epidermis to form a fluid seal over the abdominal cavity.

7. An open-cavity, reduced-pressure treatment system for providing reduced-pressure treatment within a body cavity of a patient, the system comprising:
a treatment device (10, 102, 302, 502) for disposing within the body cavity, the treatment device (10, 102, 302, 502) comprising:
a plurality of encapsulated members (106, 406), each encapsulated member (106, 406) comprising:
a manifold member (160, 508),
a first encapsulating member (168, 510) having a first side (188) and a second, patient-facing side (189,589), and
a second encapsulating member (170, 304, 512) having a first side (190, 590) and a second, patient-facing side (191), wherein fenestrations (116, 305) are formed on the second encapsulating member (170, 304, 512), and wherein the first encapsulating member (168, 510) and second encapsulating member (168, 510) encapsulate the manifold member (160, 508), and
a first reduced-pressure interface fluidly coupled to the manifold member of each of the plurality of encapsulated members (106, 406) for delivering reduced pressure to the manifold member (160, 508);
**characterized by** a sizing tool (404) for simultaneously sealing and cutting the treatment device (10, 102, 302, 502) wherein the sizing tool is adapted to size the treatment device (10, 102, 302, 502) to form a fitted treatment device;
a sealing member (124) for disposing on a portion of a patient's epidermis to form a fluid seal over the treatment device (10, 102, 302, 502) and the body cavity;
a reduced-pressure delivery conduit (130);
a reduced-pressure source fluidly coupled to the reduced-pressure delivery conduit (130); and
a second reduced-pressure interface for coupling to the sealing member and adapted to fluidly couple the reduced-pressure delivery conduit to the first reduced-pressure interface.

8. The reduced-pressure treatment system of claim 7, wherein:
the manifold member (160, 508) has a first side, a second, inward-facing side, a first lateral edge, and a second lateral edge; and
the first encapsulating member (168, 510) is disposed proximate the first side of the manifold member (160, 508), the second encapsulating member is disposed proximate the second, inward-facing side of the manifold member (160, 508, and the first encapsulating member (168, 510) and the second encapsulating member (170, 304, 512) are coupled proximate the first lateral edge and the second lateral edge of the manifold member (160, 508).

9. The reduced-pressure treatment system of claims 7 or 8, wherein:
the first encapsulating member (168, 510) has a first thickness (t₁), the second encapsulating member (170, 304, 512) has a second thickness (t₂), the manifold member has a compressed thickness (t₃); and
the sizing tool (404) comprises scissors with a blade clearance (bc) that is sized such that bc<0.9*(t₁+t₂+t₃) and bc>0.1 *(t₁+t₂+t₃).

10. The reduced-pressure treatment system of claim 7, claim8, or claim 9, wherein the sizing tool (404) comprises scissors with a blade clearance of at least 0.3 mm.

11. The reduced-pressure treatment system of claim 7 or any of claims 8-10, further comprising:
a first bonding layer (526) on the second, patient-facing side (589) of the first encapsulating member (510);
a second bonding layer (528) on the first side (590) of the second encapsulating member (512); and
wherein the sizing step further comprises causing the first bonding layer (526) and the second bonding layer to come into contact and form a bond.

12. The reduced-pressure treatment system of claim 11 or any of claims 8-10, wherein the first bonding layer (526) is a chemical bond layer and the second bonding layer (528) is a chemical bond layer, and wherein the bond is a chemical bond.

13. The reduced-pressure treatment system of claim 11, wherein the first bonding layer (526) is a pressure-sensitive adhesive layer and the second bonding layer (528) is a pressure-sensitive adhesive layer, and wherein the bond is a physical bond.

14. The reduced-pressure treatment system of claim 7 or claims 9-13, wherein:
the manifold member (160, 508) comprises reticulated foam and has a first side, a second, patient-facing side, a first lateral edge, and a second lateral edge;
the first encapsulating member (168, 510) is disposed proximate the first side of the manifold member (160, 508), the second encapsulating member (170, 304, 512) is disposed proximate the second, patient-facing side of the manifold member (160, 508), and the first encapsulating member (168, 510) and the second encapsulating member (170, 304, 512) are coupled proximate the first lateral edge and the second lateral edge of the manifold member (160, 508) to form an encapsulated member (106,406); and
the treatment device further comprises:
a central connection member (112), wherein the central connection member (112) comprises a connection manifold member (154) and wherein the plurality of encapsulated members (106, 406) are fluidly coupled to the connection manifold member (154), the connection manifold member (154) having a first side and a second, patient-facing side.

15. The reduced-pressure system of claim 14 further comprising at least one bonding layer disposed between the first encapsulating member (168, 510) and the second encapsulating member (170, 304, 512).

## Patentansprüche

1. Verfahren zum Größenanpassen einer Vorrichtung für eine Niederdruckbehandlung (10, 102, 302, 502) zum Einsetzen in eine Körperhöhle eines Patienten, wobei das Verfahren Folgendes umfasst:
Bereitstellen der Vorrichtung für eine Niederdruckbehandlung (10, 102) zum Anordnen innerhalb der Körperhöhle, wobei die Vorrichtung für eine Niederdruckbehandlung (10, 102, 302, 502) Folgendes umfasst:
ein Verteilerelement (160, 508),
ein erstes verkapselndes Element (168, 510) mit einer ersten Seite (188) und einer zweiten, dem Patienten zugewandten Seite (189,589),
ein zweites Verkapselungselement (170, 304, 512) mit einer ersten Seite (190,590) und einer zweiten, dem Patienten zugewandten Seite (191), wobei Fensteranordnungen (116, 305) in dem zweiten Verkapselungselement (170) ausgebildet sind,
wobei das erste Verkapselungselement (168) und das zweite Verkapselungselement (170) das Verteilerelement (160) verkapseln, und
eine erste Niederdruck-Kopplungsstelle, die mit dem Verteilerelement (160, 508)in Fluidverbindung steht, um das Verteilerelement (160) mit einem verringerten Druck zu beaufschlagen; und
**gekennzeichnet durch** das Größenanpassen der Vorrichtung für eine Niederdruckbehandlung (10, 102), um eine eingepasste Behandlungsvorrichtung, die in die Körperhöhle passt, unter Verwendung eines Größenanpassungswerkzeugs (404) auszubilden, wobei der Größenanpassungsschritt Folgendes umfasst:
Abdichten der Behandlungsvorrichtung (10, 102, 302) entlang einer Anpassungskante und gleichzeitig Schneiden der Behandlungsvorrichtung (10, 102, 302) entlang der Anpassungskante.

2. Verfahren zum Größenanpassen einer Vorrichtung für eine Niederdruckbehandlung (10, 102, 302, 502) nach Anspruch 1, wobei die Schritte des Abdichtens der Behandlungsvorrichtung (10, 102, 302) und des Schneidens der Behandlungsvorrichtung (10, 102, 302) die Bildung einer Trennschleifkante umfassen.

3. Verfahren zum Größenanpassen einer Vorrichtung für eine Niederdruckbehandlung (10, 102, 302, 502) nach Anspruch 1 oder 2, wobei:
das Verteilerelement (160, 508) eine erste Seite, eine zweite, nach innen weisende Seite, eine erste laterale Kante und eine zweite laterale Kante aufweist; und
das erste Verkapselungselement (168, 510) nahe der ersten Seite des Verteilerelements (160, 508) angeordnet ist, das zweite Verkapselungselement (170, 304, 512) nahe der zweiten, nach innen weisenden Seite des Verteilerelements (160, 508) angeordnetist und das erste Verkapselungselement (168, 510) und das zweite Verkapselungselement (170, 304, 512) nahe der ersten lateralen Kante und der zweiten lateralen Kante des Verteilerelements (160, 508) verbunden sind.

4. Verfahren zum Größenanpassen einer Vorrichtung für eine Niederdruckbehandlung (502) nach Anspruch 1 oder 3, des Weiteren umfassend:
eine erste Klebeschicht (526) auf der zweiten, dem Patienten zugewandten Seite (589) des ersten Verkapselungselements (510);
eine zweite Klebeschicht (528) auf der ersten Seite (590) des zweiten Verkapselungselements (512); und
wobei der Größenanpassungsschritt des Weiteren umfasst, die erste Klebeschicht (526) und die zweite Klebeschicht (528) in Kontakt miteinander zu bringen und eine Klebeverbindung zu bilden.

5. Verfahren zum Größenanpassen einer Vorrichtung für eine Niederdruckbehandlung (10, 102, 302, 502) nach Anspruch 1, 2 oder 4, wobei:
das Verteilerelement (160, 508) einen vernetzten Schaum umfasst und eine erste Seite, eine zweite, dem Patienten zugewandte Seite, eine erste laterale Kante und eine zweite laterale Kante aufweist;
das erste Verkapselungselement (168, 510) nahe der ersten Seite des Verteilerelements (160, 508) angeordnet ist, das zweite Verkapselungselement (170, 304, 512) nahe der zweiten, dem Patienten zugewandten Seite des Verteilerelements (168, 510) angeordnet ist und das erste Verkapselungselement (168, 510) und das zweite Verkapselungselement (170, 304, 512) nahe der ersten lateralen Kante und der zweiten lateralen Kante des Verteilerelements (160, 508) verbunden sind, um ein verkapseltes Element (106, 406) zu bilden;
wobei die Behandlungsvorrichtung des Weiteren Folgendes umfasst:
mehrere verkapselte Elemente (106, 406), wobei jedes verkapselte Element (106, 406) Fensteranordnungen (116) aufweist, um einem Fluid ein Strömen zwischen einer äußeren Oberfläche des verkapselten Elements zu erlauben, und
ein zentrales Verbindungselement (112), wobei das zentrale Verbindungselement (112) ein Verbindungsverteilerelement (154) umfasst, und wobei die mehreren verkapselten Elemente (106, 406) mit dem Verbindungsverteilerelement (112) in Fluidverbindung stehen, wobei das Verbindungsverteilerelement (112) eine erste Seite und eine zweite, dem Patienten zugewandte Seite aufweist, und
wobei der Größenanpassungsschritt des Weiteren das Verwenden des Größenanpassungswerkzeugs (404) umfasst, um entlang der Anpassungskante der Vorrichtung für eine Niederdruckbehandlung zu schneiden und das Verteilerelement der Vorrichtung für eine Niederdruckbehandlung innerhalb eines inneren Teils im Wesentlichen abzudichten.

6. Verfahren zum Größenanpassen einer Vorrichtung für eine Niederdruckbehandlung (10, 102, 302, 502) nach Anspruch 1, des Weiteren umfassend die folgenden Schritte:
Platzieren eines Teils der angepassten Behandlungsvorrichtung (10, 102, 302, 502) in der Körperhöhle;
Herstellen einer Fluidverbindung zwischen einer Niederdruck-Quelle und dem Verteilerelement (160, 508) und
Platzieren eines Abdichtungselements (124) auf einem Teil der Epidermis eines Patienten, um über der Bauchhöhle eine Fluidabdichtung zu bilden.

7. System für eine Niederdruckbehandlung an einer offenen Höhle, um innerhalb einer Körperhöhle eines Patienten eine Niederdruckbehandlung bereitzustellen, wobei das System Folgendes umfasst:
eine Behandlungsvorrichtung (10, 102, 302, 502) zum Einbringen in die Körperhöhle, wobei die Behandlungsvorrichtung (10, 102, 302, 502) Folgendes umfasst:
mehrere verkapselte Elemente (106, 406), wobei jedes verkapselte Element (106, 406) Folgendes umfasst:
ein Verteilerelement (160, 508),
ein erstes verkapselndes Element (168, 510) mit einer ersten Seite (188) und einer zweiten, dem Patienten zugewandten Seite (189, 589), und
ein zweites Verkapselungselement (170, 304, 512) mit einer ersten Seite (190, 590) und einer zweiten, dem Patienten zugewandten Seite (191), wobei Fensteranordnungen (116, 305) in dem zweiten Verkapselungselement (170, 304, 512) ausgebildet sind, und wobei das erste Verkapselungselement (168, 510) und das zweite Verkapselungselement (168, 510) das Verteilerelement (160, 508) verkapseln; und
eine erste Niederdruck-Kopplungsstelle, die mit dem Verteilerelement von jedem der mehreren verkapselten Elemente (106, 406) in Fluidverbindung steht, um am Verteilerelement (160, 508) einen verringerten Druck zu liefern;
**gekennzeichnet durch** ein Größenanpassungswerkzeug (404) zum gleichzeitigen Abdichten und Schneiden der Behandlungsvorrichtung (10, 102, 302, 502) wobei das Größenanpassungswerkzeug geeignet ist, um die Größe der Behandlungsvorrichtung (10, 102, 302, 502) anzupassen, um eine eingepasste Behandlungsvorrichtung zu bilden;
ein Abdichtungselement (124) zum Anbringen auf einem Teil der Epidermis eines Patienten, um eine Fluidabdichtung über der Behandlungsvorrichtung (10, 102, 302, 502) und der Körperhöhle zu bilden;
eine Niederdruck-Abgabeleitung (130);
eine Niederdruck-Quelle, die mit der Niederdruck-Abgabeleitung (130) in Fluidverbindung steht; und
eine zweite Niederdruck-Kopplungsstelle zum Verbinden mit dem Abdichtungselement, die geeignet ist, um eine Fluidverbindung zwischen der Niederdruck-Abgabeleitung und der ersten Niederdruck-Kopplungsstelle herzustellen.

8. System zur Niederdruckbehandlung nach Anspruch 7, wobei:
das Verteilerelement (160, 508) eine erste Seite, eine zweite, nach innen weisende Seite, eine erste laterale Kante und eine zweite laterale Kante aufweist; und
das erste Verkapselungselement (168, 510) naheder ersten Seite des Verteilerelements (160, 508) angeordnet ist, das zweite Verkapselungselement nahe der zweiten, nach innen weisenden Seite des Verteilerelements (160, 508) angeordnet ist und das erste Verkapselungselement (168, 510) und das zweite Verkapselungselement (170, 304, 512) naheder ersten lateralen Kante und der zweiten lateralen Kante des Verteilerelements (160, 508) verbunden sind.

9. System zur Niederdruckbehandlung nach den Ansprüchen 7 oder 8, wobei:
das erste Verkapselungselement (168, 510) eine erste Dicke (t₁) aufweist, das zweite Verkapselungselement (170, 304, 512) eine zweite Dicke (t₂) aufweist, das Verteilerelement eine komprimierte Dicke (t₃) aufweist, und
das Größenanpassungswerkzeug (404) eine Schere mit einem Schneidspalt (bc) aufweist, der so bemessen ist, dass bc < 0,9*(t₁+t₂+t₃) und bc > 0,1*(t₁+t₂+t₃) ist.

10. System zur Niederdruckbehandlung nach Anspruch 7, 8 oder 9, wobei das Größenanpassungswerkzeug (404) eine Schere mit einem Schneidspalt von mindestens 0,3 mm umfasst.

11. System zur Niederdruckbehandlung nach Anspruch 7 oder einem der Ansprüche 8-10, des Weiteren umfassend:
eine erste Klebeschicht (526) auf der zweiten, dem Patienten zugewandten Seite (589) des ersten Verkapselungselements (510);
eine zweite Klebeschicht (528) auf der ersten Seite (590) des zweiten Verkapselungselements (512); und
wobei der Größenanpassungsschritt des Weiteren umfasst, die erste Klebeschicht (526) und die zweite Klebeschicht in Kontakt miteinander zu bringen und eine Klebeverbindung zu bilden.

12. System zur Niederdruckbehandlung nach Anspruch 11 oder einem der Ansprüche 8-10, wobei die erste Klebeschicht (526) eine chemische Klebeschicht ist und die zweite Klebeschicht (528) eine chemische Klebeschicht ist, und wobei die Klebeverbindung eine chemische Bindung ist.

13. System zur Niederdruckbehandlung nach Anspruch 11, wobei die erste Klebeschicht (526) eine drucksensitive Haftschicht ist und die zweite Klebeschicht (528) eine drucksensitive Haftschicht ist, und wobei die Klebeverbindung eine physikalische Klebeverbindung ist.

14. System zur Niederdruckbehandlung nach Anspruch 7 oder den Ansprüchen 9-13, wobei:
das Verteilerelement (160, 508) einen vernetzten Schaum umfasst und eine erste Seite, eine zweite, dem Patienten zugewandte Seite, eine erste laterale Kante und eine zweite laterale Kante aufweist;
das erste Verkapselungselement (168, 510) nahe der ersten Seite des Verteilerelements (160, 508) angeordnet ist, das zweite Verkapselungselement (170, 304, 512) nahe der zweiten, dem Patienten zugewandten Seite des Verteilerelements (168, 508) angeordnet ist und das erste Verkapselungselement (168, 510) und das zweite Verkapselungselement (170, 304, 512) nahe der ersten lateralen Kante und der zweiten lateralen Kante des Verteilerelements (160, 508) verbunden sind, um ein verkapseltes Element (106, 406) zu bilden; und
wobei die Behandlungsvorrichtung des Weiteren Folgendes umfasst:
ein zentrales Verbindungselement (112), wobei das zentrale Verbindungselement (112) ein Verbindungsverteilerelement (154) umfasst, und wobei die mehreren verkapselten Elemente (106, 406) mit dem Verbindungsverteilerelement (154) in Fluidverbindung stehen, wobei das Verbindungsverteilerelement (154) eine erste Seite und eine zweite, dem Patienten zugewandte Seite aufweist.

15. Niederdruck-System nach Anspruch 14, des Weiteren umfassend mindestens eine Klebeschicht, die zwischen dem ersten Verkapselungselement (168, 510) und dem zweiten Verkapselungselement (170, 304, 512) angeordnet ist.

## Revendications

1. Procédé de dimensionnement d'un dispositif de traitement à pression réduite (10, 102, 302, 502) pour un placement dans une cavité corporelle d'un patient, le procédé comprenant les étapes consistant à :
fournir le dispositif de traitement à pression réduite (10, 102) pour le disposer à l'intérieur de la cavité corporelle, dans lequel le dispositif de traitement à pression réduite (10, 102, 302, 502) comprend :
un élément collecteur (160, 508),
un premier élément d'encapsulation (168, 510) ayant un premier côté (188) et un second côté (189, 589) faisant face au patient,
un second élément d'encapsulation (170, 304, 512) ayant un premier côté (190, 590) et un second côté (191) faisant face au patient, dans lequel des fenêtrages (116, 305) sont formés dans le second élément d'encapsulation (170),
dans lequel le premier élément d'encapsulation (168) et le second élément d'encapsulation (170) encapsulent l'élément collecteur (160), et
une première interface à pression réduite en couplage fluidique avec l'élément collecteur (160, 508) pour délivrer une pression réduite à l'élément collecteur (160) ; et
**caractérisé par** le dimensionnement du dispositif de traitement à pression réduite (10, 102) pour former un dispositif de traitement ajusté qui adapte la cavité corporelle en utilisant un outil de dimensionnement (404), l'étape de dimensionnement comprenant :
le scellage étanche du dispositif de traitement (10, 102, 302) le long d'un bord d'ajustement et la découpe simultanée du dispositif de traitement (10, 102, 302) le long du bord d'ajustement.

2. Procédé de dimensionnement du dispositif de traitement à pression réduite (10, 102, 302, 502) selon la revendication 1, dans lequel les étapes de scellage étanche du dispositif de traitement (10, 102, 302) et de découpe du dispositif de traitement (10, 102, 302) comprennent la formation d'une découpe par friction.

3. Procédé de dimensionnement du dispositif de traitement à pression réduite (10, 102, 302, 502) selon les revendications 1 ou 2, dans lequel :
l'élément collecteur (160, 508) a un premier côté, un second côté tourné vers l'intérieur, un premier bord latéral et un second bord latéral ; et
le premier élément d'encapsulation (168, 510) est disposé à proximité du premier côté de l'élément collecteur (160, 508), le second élément d'encapsulation (170, 304, 512) est disposé à proximité du second côté tourné vers l'intérieur de l'élément collecteur (160, 508) et le premier élément d'encapsulation (168, 510) et le second élément d'encapsulation (170, 304, 512) sont couplés à proximité du premier bord latéral et du second bord latéral de l'élément collecteur (160, 508).

4. Procédé de dimensionnement du dispositif de traitement à pression réduite (502) selon la revendication 1 ou la revendication 3, comprenant en outre :
une première couche de liaison (526) sur le second côté (589) tourné vers le patient du premier élément d'encapsulation (510) ;
une seconde couche de liaison (528) sur le premier côté (590) du second élément d'encapsulation (512) ; et
dans lequel l'étape de dimensionnement comprend en outre le fait d'amener la première couche de liaison (526) et la seconde couche de liaison (528) à venir en contact et à former une liaison.

5. Procédé de dimensionnement du dispositif de traitement à pression réduite (10, 102, 302, 502) selon la revendication 1, 2 ou 4, dans lequel :
l'élément collecteur (160, 508) comprend de la mousse réticulée et a un premier côté, un second côté tourné vers le patient, un premier bord latéral et un second bord latéral ;
le premier élément d'encapsulation (168, 510) est disposé à proximité du premier côté de l'élément collecteur (160, 508), le second élément d'encapsulation (170, 304, 512) est disposé à proximité du second côté tourné vers le patient de l'élément collecteur (168, 510) et le premier élément d'encapsulation (168, 510) et le second élément d'encapsulation (170, 304, 512) sont couplés à proximité du premier bord latéral et du second bord latéral de l'élément collecteur (160, 508) pour former un élément encapsulé (106, 406) ;
le dispositif de traitement comprend en outre :
une pluralité d'éléments encapsulés (106, 406), chaque élément encapsulé (106, 406) ayant des fenêtrages (116) pour permettre l'écoulement de fluide entre une surface externe de l'élément encapsulé et
un élément de raccordement central (112), dans lequel l'élément de raccordement central (112) comprend un élément collecteur de raccordement (154) et dans lequel la pluralité d'éléments encapsulés (106, 406) sont en communication fluidique avec l'élément collecteur de raccordement (112), l'élément collecteur de raccordement (112) ayant un premier côté et un second côté tourné vers le patient ; et
l'étape de dimensionnement comprend en outre l'utilisation de l'outil de dimensionnement (404) pour découper le long du bord d'ajustement du dispositif de traitement à pression réduite et sceller sensiblement l'élément collecteur du dispositif de traitement à pression réduite dans une portion intérieure.

6. Procédé de dimensionnement du dispositif de traitement à pression réduite (10, 102, 302, 502) selon la revendication 1, comprenant en outre les étapes consistant à :
placer une partie du dispositif de traitement ajusté (10, 102, 302, 502) dans la cavité corporelle ;
établir une communication fluidique d'une source à pression réduite avec l'élément collecteur (160, 508) ; et
placer un élément de scellage étanche (124) sur une partie de l'épiderme d'un patient afin de former un joint étanche aux fluides sur la cavité abdominale.

7. Système de traitement à pression réduite et à cavité ouverte pour assurer un traitement à pression réduite dans une cavité corporelle d'un patient, le système comprenant :
un dispositif de traitement (10, 102, 302, 502) à disposer dans la cavité corporelle, le dispositif de traitement (10, 102, 302, 502) comprenant :
une pluralité d'éléments encapsulés (106, 406), chaque élément encapsulé (106, 406) comprenant :
un élément collecteur (160, 508),
un premier élément d'encapsulation (168, 510) ayant un premier côté (188) et un second côté (189, 589) tourné vers le patient, et
un second élément d'encapsulation (170, 304, 512) ayant un premier côté (190, 590) et un second côté (191) tourné vers le patient, dans lequel des fenêtrages (116, 305) sont formés sur le second élément d'encapsulation (170, 304, 512) et dans lequel le premier élément d'encapsulation (168, 510) et le second élément d'encapsulation (168, 510) encapsulent l'élément collecteur (160, 508) et
une première interface à pression réduite en communication fluidique avec l'élément collecteur de chacun de la pluralité d'éléments encapsulés (106, 406) pour délivrer une pression réduite à l'élément collecteur (160, 508) ;
**caractérisé par** un outil de dimensionnement (404) pour sceller et découper simultanément le dispositif de traitement (10, 102, 302, 502), dans lequel l'outil de dimensionnement est à même de dimensionner le dispositif de traitement (10, 102, 302, 502) pour former un dispositif de traitement ajusté ;
un élément de scellage étanche (124) pour disposer sur une partie de l'épiderme d'un patient afin de former un joint étanche aux fluides sur le dispositif de traitement (10, 102, 302, 502) et la cavité corporelle ;
un conduit de délivrance de pression réduite (130) ;
une source à pression réduite en communication fluidique avec le conduit de délivrance de pression réduite (130) ; et
une seconde interface à pression réduite pour coupler à l'élément de scellage étanche et qui est à même d'établir un couplage fluidique entre le conduit de délivrance de pression réduite et la première interface de pression réduite.

8. Système de traitement à pression réduite selon la revendication 7, dans lequel :
l'élément collecteur (160, 508) a un premier côté, un second côté tourné vers l'intérieur, un premier bord latéral et un second bord latéral ; et
le premier élément d'encapsulation (168, 510) est disposé à proximité du premier côté de l'élément collecteur (160, 508), le second élément d'encapsulation est disposé à proximité du second côté tourné vers l'intérieur de l'élément collecteur (160, 508) et le premier élément d'encapsulation (168, 510) et le second élément d'encapsulation (170, 304, 512) sont couplés à proximité du premier bord latéral et du second bord latéral de l'élément collecteur (160, 508).

9. Système de traitement à pression réduite selon la revendication 7 ou la revendication 8, dans lequel :
le premier élément d'encapsulation (168, 510) a une première épaisseur (t₁), le second élément d'encapsulation (170, 304, 512) a une seconde épaisseur (t₂), l'élément collecteur a une épaisseur à l'état comprimé (t₃) ; et
l'outil de dimensionnement (404) comprend des ciseaux avec un jeu entre les lames (bc) qui est dimensionné de sorte que bc<0, 9* (t₁+t₂+t₃) et bc>0, 1* (t₁+t₂+t₃).

10. Système de traitement à pression réduite selon la revendication 7, la revendication 8 ou la revendication 9, dans lequel l'outil de dimensionnement (404) comprend des ciseaux avec un jeu entre les lames d'au moins 0,3 mm.

11. Système de traitement à pression réduite selon la revendication 7 ou l'une quelconque des revendications 8 à 10, comprenant en outre :
une première couche de liaison (526) sur le second côté (589) tourné vers le patient du premier élément d'encapsulation (510) ;
une seconde couche de liaison (528) sur le premier côté (590) du second élément d'encapsulation (512) ; et
dans lequel l'étape de dimensionnement comprend en outre le fait d'amener la première couche de liaison (526) et la seconde couche de liaison à venir en contact et à former une liaison.

12. Système de traitement à pression réduite selon la revendication 11 ou l'une quelconque des revendications 8 à 10, dans lequel la première couche de liaison (526) est une couche de liaison chimique et la seconde couche de liaison (528) est une couche de liaison chimique et dans lequel la liaison est une liaison chimique.

13. Système de traitement à pression réduite selon la revendication 11, dans lequel la première couche de liaison (526) est une couche d'adhésif sensible à la pression et la seconde couche de liaison (528) est une couche d'adhésif sensible à la pression et dans lequel la liaison est une liaison physique.

14. Système de traitement à pression réduite selon la revendication 7 ou selon les revendications 9 à 13, dans lequel :
l'élément collecteur (160, 508) comprend de la mousse réticulée et a un premier côté, un second côté tourné vers le patient, un premier bord latéral et un second bord latéral ;
le premier élément d'encapsulation (168, 510) est disposé à proximité du premier côté de l'élément collecteur (160, 508), le second élément d'encapsulation (170, 304, 512) est disposé à proximité du second côté tourné vers le patient de l'élément collecteur (160, 508) et le premier élément d'encapsulation (168, 510) et le second élément d'encapsulation (170, 304, 512) sont couplés à proximité du premier bord latéral et du second bord latéral de l'élément collecteur (160, 508) pour former un élément encapsulé (106, 406) ; et
le dispositif de traitement comprend en outre :
un élément de raccordement central (112), dans lequel l'élément de raccordement central (112) comprend un élément collecteur de raccordement (154) et dans lequel la pluralité d'éléments encapsulés (106, 406) sont en communication fluidique avec l'élément collecteur de raccordement (154), l'élément collecteur de raccordement (154) ayant un premier côté et un second côté tourné vers le patient.

15. Système à pression réduite selon la revendication 14, comprenant en outre au moins une couche de liaison disposée entre le premier élément d'encapsulation (168, 510) et le second élément d'encapsulation (170, 304, 512).
